# EUROPEAN PATENT SPECIFICATION

(11) **EP 0 398 218 B1**
(45) Date of publication and mention of the grant of the patent: **04.01.1995**
(21) Application number: 90109035.7
(22) Date of filing: 14.05.1990
(51) Int. Cl.: A61B 17/22, A61B 6/10

(54) **Acoustic wave therapy apparatus**
Gerät für Schallwellentherapie
Appareil de thérapie par ondes acoustiques

(30) Priority: 15.05.1989 JP 121207/89
(43) Date of publication of application: 22.11.1990
(73) Proprietor: KABUSHIKI KAISHA TOSHIBA, Kawasaki-shi, Kanagawa-ken 210 (JP)
(72) Inventor: Nomura, Satoshi, c/o Intellectual Poperty Div., Minato-ku, Tokyo 105 (JP)
(74) Representative: Blumbach, Kramer & Partner

(56) References cited:
- EP-A- 0 296 349
- EP-A- 0 301 198
- EP-A- 0 301 360
- DE-A- 3 532 184

## Description

The present invention relates to an acoustic wave therapy apparatus according to the pre-characterizing part of claim 1. The apparatus is suitable for treating cancerous cells or calculi in a human body, using shock waves produced by acoustic waves or continuous acoustic waves. More particularly, the invention relates to an ultrasonic shock wave lithotrity apparatus and an ultrasonic continuous wave hyperthermic therapy apparatus.

Conventional ultrasonic treatment apparatuses include an ultrasonic shock wave lithotrity apparatus for breaking calculi, etc. in a human body, using shock waves due to focusing ultrasonic energy, and an ultrasonic continuous wave hyperthermic therapy apparatus for treating a tumor by heating it up to a lethal temperature, using continuous focusing ultrasonic energy.

This type of ultrasonic treatment apparatus generally has an acoustic wave generator for generating ultrasonic waves. The acoustic wave generator has a concave surface for focusing the generated ultrasonic waves. The acoustic wave generator is arranged to face a subject, with a bag interposed therebetween. An ultrasonic imaging probe is arranged within the bag, for positioning the acoustic wave generator and for obtaining tomograms of the subject for confirming the result of treatment.

If a large-diameter acoustic wave generator is employed in this apparatus and generated waves are focused onto a region-of-interest (ROI), a maximum treatment effect is attained with a small treatment energy. In this case, however, if the focus of waves departs from the ROI, a normal tissue is damaged. It is thus important to precisely focus the waves onto the ROI.

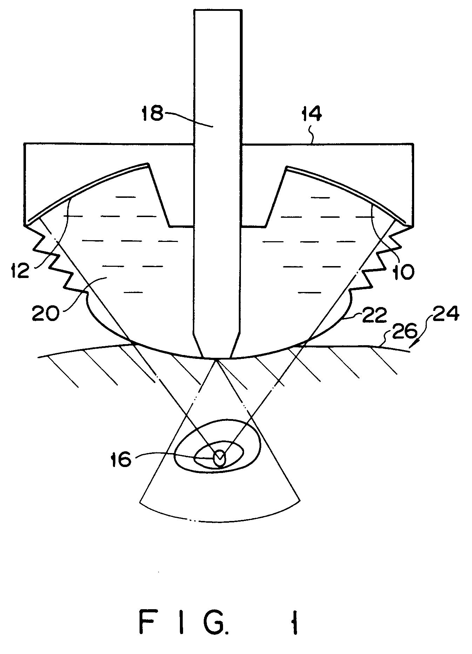

Fig. 1 is a cross-sectional view for schematically showing a structure of an acoustic wave therapy apparatus according to the pre-characterizing part of claim 1. In Fig. 1, irradiation devices (as the acoustic wave generator) 10 and 12 serving as acoustic wave generators are secured to a holder 14. The irradiation devices 10 and 12 are arranged symmetrical in respect of an ultrasonic imaging probe 18 (described later) so that the focal points of the devices 10 and 12 may be positioned at a region-of-interest (ROI) 16. The acoustic wave therapy apparatus has a bag member 22 containing water 20 which is put in close contact with a surface 26 of a subject 24. The ultrasonic imaging probe 18 is arranged at the center of the holder 14 and is employed for effecting positioning for treatment and for receiving ultrasonic waves reflected from the ROI 16, thereby confirming if the ROI 16 has been treated appropriately. In Fig. 1, a dot-and-dash line indicates a region where ultrasonic waves for treatment are radiated, and a two-dot-and-dash line indicates a region where ultrasonic waves for ultrasonic imaging treatment are radiated.

When an acoustic wave therapy is performed by this apparatus, ultrasonic waves are radiated onto the ROI 16 in the subject 24 and the ultrasonic imaging probe 18 receives the waves reflected by the ROI 16. Tomograms are formed on the basis of the reflected waves, and the formed tomograms are displayed. Observing the tomograms, an operator moves and positions the apparatus so that the focal points of the irradiation devices 10 and 12 may coincide with the location of the ROI 16. When the focal points of the devices 10 and 12 have coincided with the location of the ROI 16, the devices 10 and 12 are driven to radiate intense ultrasonic waves onto the ROI 16, thereby treating the ROI 16.

As has been stated above, the ultrasonic imaging probe 18 is put on the surface 26 of the subject 24 via the water bag 22, manually or by means of an electric mechanism, thereby focusing the waves radiated from the devices 10 and 12 at the ROI 16 and confirming the result of treatment.

In the above-described prior art, however, the operator is unable to sense the intensity of the pressure under which the imaging probe 18 is pressed on the subject 24. Thus, if the operator presses the imaging probe 18 under high pressure for obtaining better tomograms, there is a concern that the subject may be injured.

EP-A1-0 301 360 discloses a lithotrity apparatus in which the imaging probe can be rotated by means of a driving mechanism. The probe can also be moved vertically. EP-A1- 0 301 198 shows a lithotrity apparatus having a collision preventing means, which is constituted by a hose filled with a liquid. When the hose interferes with the body of a patient, the pressure of the liquid is increased. The increased pressure is detected by a pressure switch.

The object of the present invention is to provide an acoustic wave therapy apparatus ensuring the safety for a subject.

This object is achieved by the invention according to the characterizing features of claim 1.

This invention can be more fully understood from the following detailed description when taken in conjunction with the accompanying drawings, in which:
Fig. 1 is a cross-sectional view for schematically showing a structure of a conventional acoustic wave therapy apparatus;
Fig. 2 is a cross-sectional view for schematically showing a structure of an acoustic wave therapy apparatus according to an embodiment of the present invention;
Fig. 3 is a perspective view for showing a mechanical section mounted on a holder in the apparatus shown in Fig. 2;
Fig. 4 is a block diagram for illustrating an electric circuit in the case where the apparatus shown in Fig. 1 is employed as an ultrasonic shock wave lithotrity apparatus;
Fig. 5 is a block diagram for illustrating an electric circuit in the case where the apparatus shown in Fig. 1 is employed as an ultrasonic continuous wave hyperthermic therapy apparatus; and
Fig. 6 is a cross-sectional view for schematically showing a structure of an acoustic wave therapy apparatus according to another embodiment of the invention.

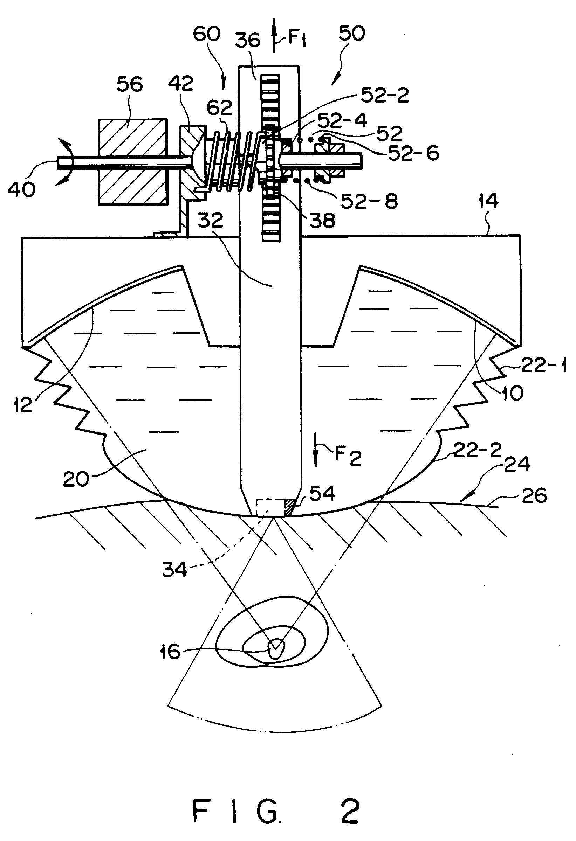

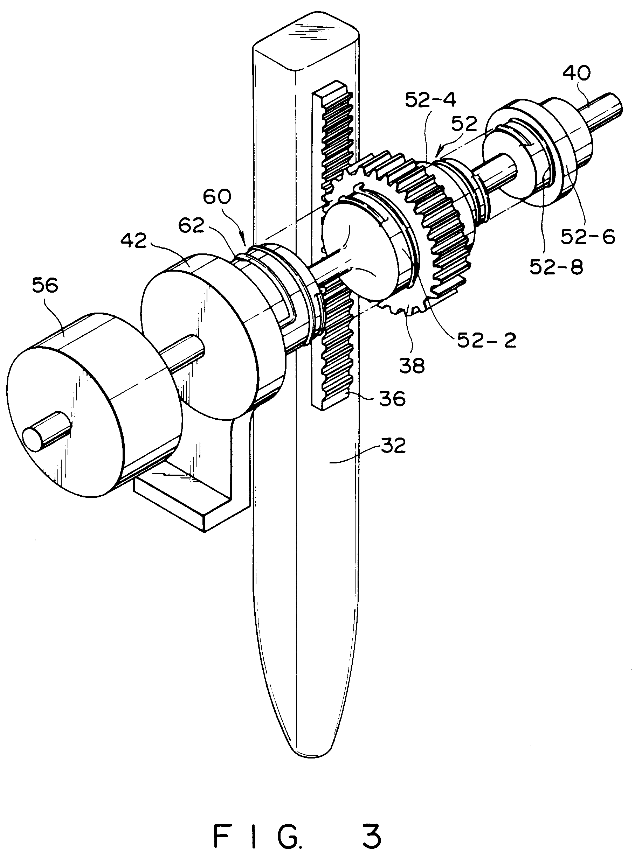

An embodiment of the present invention will now be described with reference to Figs. 2 and 3.

In an acoustic wave therapy apparatus shown in Fig. 2, irradiation devices (as the acoustic wave generator) 10 and 12 for generating intense ultrasonic pulse waves or intense continuous ultrasonic waves are arranged on one side of a holder 14. A bellows 22-1 and a water bag 22-2, which constitute a bag member, are attached to the oscillators 10 and 12. The water bag 22-2 is put in contact with a surface 26 of a subject 24.

An advanceable/retractable probe rod 32 is arranged in the water bag 22, passing through a hole formed in the holder 14. An ultrasonic probe 34 for ultrasonic tomographic imaging is provided at an end portion of the probe rod 32.

As is shown in Figs. 2 and 3, the probe rod 32 is provided with a rack gear 36 extending in the longitudinal direction of the probe rod 32. A pinion gear engaging the rack gear 36, a gear drive shaft 40, and a baring seat are arranged on the side of the holder 14, which is opposite to the side where the devices 10 and 12 are arranged.

In addition, a pressing force maintaining device 50 is provided for limiting to a predetermined value the pressure under which the end portion of the probe rod 32 is pressed on the surface 26 of the subject 24 via the water bag 22. There is also provided a retracting device 60 for retracting the probe rod 32 when the end portion of the probe rod 32 receives a predetermined pushing force from the surface 26 via the water bag 22-2.

The apparatus according to this embodiment is characterized by the pressing force maintaining device 50 and the retracting device 60. As mentioned above, the maintaining device 50 limits to a predetermined value the pressure under which the end portion of the probe rod 32 is pressed on the surface 26 of subject 24. The retracting device 60 retracts the probe rod 32 from the subject 24 when the subject 24 pushes up the probe rod 32 with a predetermined force.

The pressing force maintaining device 60 comprises a torque limiter 52, a pressure sensor 54, and a torque sensor 56.

The torque limiter 52 comprises a receiving plate 52-2 fixed on the drive shaft 40, a first plate 52-4 movably mounted on the drive shaft 40 to clamp the pinion gear between itself and the receiving plate 52-2, a second plate 52-6 fixed on the drive shaft 40 with a distance from the first plate 52-4, and a coil spring 52-8 mounted on the shaft 40 between the first plate 52-4 and the second plate 52-6. The pinion gear 38 arranged between the receiving plate 52-2 and the first plate 52-4 is clamped by means of the coil spring 52-8. This clamping force is equal to an upper limit value of the pressing force of the probe rod 32.

Thus, when the drive shaft 40 is rotated manually or by means of an electric mechanism, with a torque not higher than the upper limit value of the pressing force of the probe rod 32, the probe rod 32 can freely move vertically because the drive shaft 40, the receiving plate 52-2, the pinion gear 38, the first plate 52-4 and the second plate 52-6 rotate as one body.

On the other hand, when the drive shaft 40 is rotated with a torque higher than the upper limit value of the pressing force, the pinion gear 38 rotates idly though the drive shaft 40, the receiving plate 52-2, the first plate 52-4 and the second plate 52-6 rotate as one body.

More specifically, when the drive shaft 40 is rotated manually or by means of an electric mechanism, the torque higher than the upper limit value of the pressing force is not transmitted from the shaft 40 to the probe rod 32. Since a high pressing force is not applied to the subject 24 who is situated under the end portion of the probe rod 32, the safety for the subject 24 is ensured. For the purpose of clear understanding, the operation of the torque limiter 52 of the maintaining device 50 has been described, supposing that a torsion spring 62 in the retracting device 60 is not provided.

The pressure sensor 54 in the maintaining device 50 is provided at the end of the probe rod 32 or at the ultrasonic probe 34. The pressure sensor 54 senses the pressing force of the probe rod 32 and, when the sensed value exceeds a predetermined value, the sensor 54 indicates this fact, for example, by an alarm.

The torque sensor 56 is mounted on the drive shaft 40 and stops the rotation of the drive shaft 40 when the output from the sensor 56 exceeds a predetermined value. The maintaining device 50 may further include a strain gage on the drive shaft 40, so that the rotation of the drive shaft 40 may be stopped when the output from the strain gage exceeds a predetermined level.

In this embodiment, the retracting device 60 includes the aforementioned torsion spring 62. The torsion spring 62 is mounted on the drive shaft 40. The torsion spring 62 is fixed at one end to the bearing seat 42 and at the other end to the peripheral portion of the pinion gear 38. The torsion spring 62 lifts the probe rod 32 with an upward force F1 (indicated in Fig. 2). The upward force F1 is equivalent to the sum of the frictional force (acting upwards) between the probe rod 32 and the holder 14 and a downward force F2. The downward force F2 is produced by the weight of the probe rod 32, the ultrasonic probe 34 and the rack gear 36.

By virtue of this cancelling of upward and downward forces, the end portion of the probe rod 32 is allowed to contact with the surface 26 of the subject 24, with a suitable pressing force of, for example, 1 to 2 Kg.

The torsion spring 62 cancels the downward force F2 due to the weight of the probe rod 32, the ultrasonic probe 34 and the rack gear 36. Thus, the probe rod 32 is easily lifted by the force smaller than the force F2 and larger than the frictional force between the probe rod 32 and the holder 14, for example, when the subject 24 inhales and his/her belly swells. In this case, when no force is applied to the drive shaft 40, all the drive shaft 40, the receiving plate 52-2, the pinion gear 38, the first plate 52-4 and the second plate 52-6 rotate as one body.

According to the apparatus having the above structure, when the drive shaft 40 is rotated with a torque exceeding a predetermined value, the excessive torque is not transmitted to the probe rod 32. Thus, the subject 24 is prevented from being injured, and the ultrasonic probe 34 is brought into contact with the surface 26 of the subject 24 under a suitable pressure. Therefore, excellent images can be obtained.

The pressure sensor 54 senses the pressing force of the probe rod 32. When the force sensed by the pressure sensor 54 exceeds a predetermined value, the operator's attention drawn to this fact by an alarm.

The torque sensor 52 senses the torque of the drive shaft 40, and, when the torque of the shaft 40 exceeds a predetermined value, the rotation of the drive shaft 40 is stopped. Thus, an excessive pressing force is not applied to the subject 24, and the subject 24 is prevented from being injured.

The torsion spring 62 is able to retract the probe rod 32 from the subject 24. When the probe rod 32 is put in contact with the subject 24, the torsion spring 62 limits the force with which the rod 32 is pressed on the subject 24. When the subject 24 moves upwards while the probe rod 32 is made to contact the subject 24, the probe rod 32 moves upwards. Thus, the subject 24 is prevented from being injured, and the safety for the subject 24 is ensured.

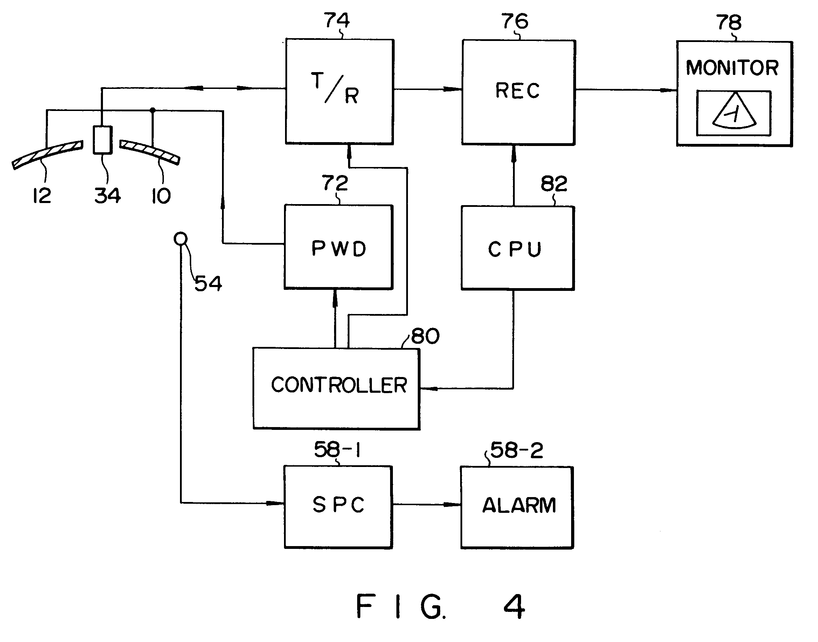

Fig. 4 is a block diagram for illustrating an electric circuit in the case where the apparatus shown in Fig. 1 is employed as an ultrasonic shock wave lithotrity apparatus. The irradiation devices 10 and 12 are driven by a pulse wave driver (PWD) 72 to radiate intense ultrasonic pulse waves. The ultrasonic probe 34 is driven by a transmitter/receiver (T/R) 74, and a received signal is sent to a reconstruction device (REC) 76, and the REC 76 produces, for example, tomograms. The produced tomograms are displayed on a monitor 78. A controller 80 controls the transmitter/receiver 74 and the pulse wave driver 72. The controller 80 receives commands from a CPU 82. The output from the pressure sensor 54 is applied to a signal processing circuit (SPC) 58-1. When the SPC 58-1 determines that the output from the pressure sensor 54 exceeds a predetermined level, an alarm 58-2 is driven to generate an alarm sound.

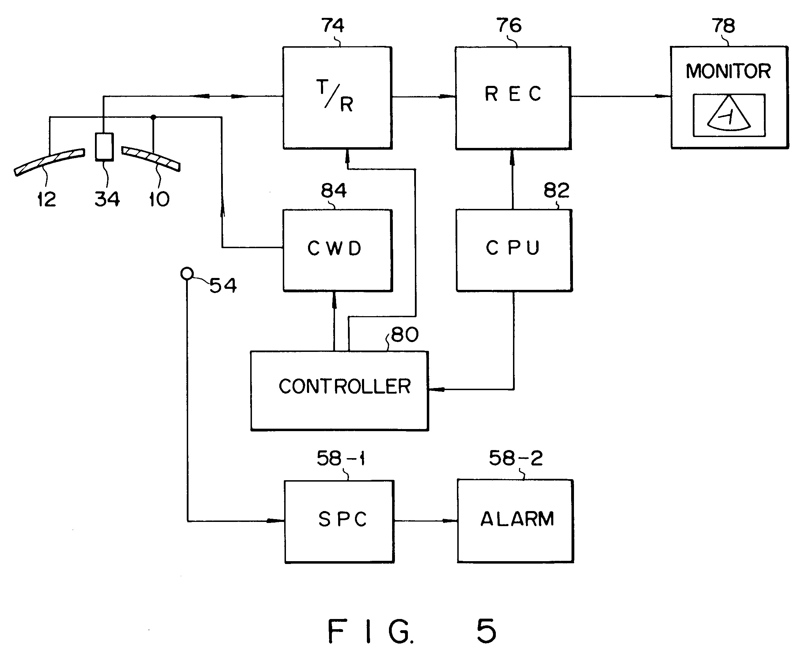

Fig. 5 is a block diagram for illustrating an electric circuit in the case where the apparatus shown in Fig. 1 is employed as an ultrasonic continuous wave hyperthermic therapy apparatus. The irradiation devices 10 and 12 are driven by a continuous wave driver (CWD) 84 to generate intense continuous ultrasonic waves. The other structure is the same as that of the apparatus illustrated in Fig. 4.

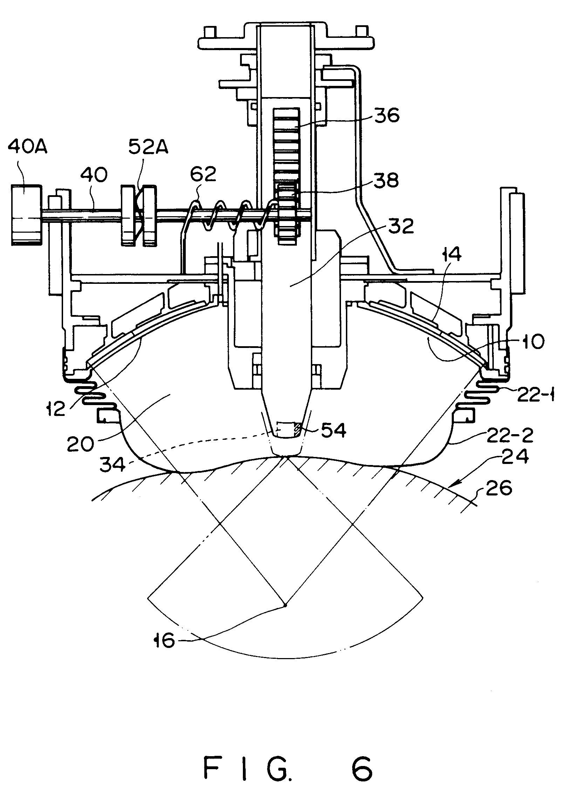

Fig. 6 is a cross-sectional view for schematically showing a structure of an acoustic wave therapy apparatus according to another embodiment of the invention. The structure of this apparatus is basically identical with that of the apparatus shown in Fig. 1 (and Fig. 2), excepting that a torque limiter 52A is provided at a middle portion of the drive shaft 40, and a manual knob 40A is provided at an end portion of the drive shaft 40.

As has been described above, the present invention provides an acoustic wave therapy apparatus ensuring the safety for the subject.

## Claims

1. An acoustic wave therapy apparatus comprising:
a holder (14) having a concave surface at least on one side thereof, and also having a through-hole at its central portion;
acoustic wave generating means (10, 12) provided on said one side of said holder (14), for generating acoustic waves for medical treatment;
a bag member (22-1, 22-2) arranged on said one side of the holder (14) and designed to be put in contact with the surface (26) of a subject, and containing an acoustic wave propagation medium;
a rod member (32) inserted into said through-hole of the holder (14) and arranged vertically movable in said bag member (22-1, 22-2); and
an ultrasonic probe (34) for ultrasonic wave tomographic imaging, provided at the bag member-side end portion of the rod member (32);
characterized in that said acoustic wave therapy apparatus comprises
a drive mechanism (36, 38, 40, 50, 60) for vertically moving the ultrasonic probe (34) relative to the holder (14); and
a torque limiter (52; 52A) provided on said drive mechanism (36, 38, 40, 50, 60), for limiting to a predetermined value the pressing force with which the rod member (32) is pressed on the surface (26).

2. The apparatus according to claim 1, characterized in that said acoustic wave generating means (10, 12) generates intense pulse waves.

3. The apparatus according to claim 1, characterized in that said acoustic wave generating means (10, 12) generates intense continuous waves.

4. The apparatus according to claim 1, characterized in that said acoustic wave propagation medium is water (20).

5. The apparatus according to claim 1, characterized in that said acoustic wave therapy apparatus further comprises a torque sensor (56) arranged on said drive mechanism (36, 38, 40, 50, 60), for sensing the torque acting on the drive mechanism and a stop mechanism for stopping the driving of said drive mechanism (36, 38, 40, 50, 60) when the output from the torque sensor (56) exceeds a predetermined level.

6. The apparatus according to claim 1, characterized in that said acoustic wave therapy apparatus comprises
retracting means (60), arranged on the other side of the holder (14), for retracting said rod member (32) when the end portion of the rod member (32) receives a predetermined upward force from the surface of the subject via the bag member (22-1, 22-2).

7. The apparatus according to claim 6, characterized in that said retracting means (60) is a torsion spring mechanism (62) mounted on said drive mechanism (36, 38, 40, 50, 60).

## Patentansprüche

1. Schallwellentherapiegerät, umfassend:
einen Halter (14), der an mindestens seiner einen Seite eine konkave Oberfläche und in seinem Mittelabschnitt außerdem ein Durchgangsloch aufweist;
eine Schallwellengeneratoreinrichtung (10, 12), die auf der einen Seite des Halters (14) vorgesehen ist, um Schallwellen für die medizinische Behandlung zu erzeugen;
ein Sackteil (22-1, 22-2), das auf der einen Seite des Halters (14) angeordnet und derart ausgebildet ist, daß es in Berührung mit der Oberfläche (26) eines Patienten bringbar ist, und das ein Schallwellenausbreitungsmedium enthält;
ein Stabelement (32), das in das Durchgangsloch des Halters (14) eingesetzt und vertikal beweglich in dem Sackteil (22-1, 22-2) angeordnet ist; und
eine Ultraschallsonde (34) für die tomographische Ultraschallwellenbildgebung, angeordnet an dem sackteilseitigen Endabschnitt des Stabelements (32);
dadurch **gekennzeichnet**, daß das Schallwellentherapiegerät aufweist
einen Antriebsmechanismus (36, 38, 40, 50, 60) zum vertikalen Bewegen der Ultraschallsonde (34) relativ zu dem Halter (14);
einen Drehmomentbegrenzer (52; 52A), der an dem Ahtriebsmechanismus (36, 38, 40, 50, 60) vorgesehen ist, um die Andrückkraft, mit welcher das Stabelement (32) gegen die Oberfläche (26) gedrückt wird, auf einen vorbestimmten Wert zu begrenzen.

2. Gerät nach Anspruch 1 dadurch **gekennzeichnet**, daß die Ultraschallwellengeneratoreinrichtung (10, 12) starke Impulswellen erzeugt.

3. Gerät nach Anspruch 1 dadurch **gekennzeichnet**, daß die Schallwellengeneratoreinrichtung (10, 12) starke kontinuierliche Wellen erzeugt.

4. Gerät nach Anspruch 1 dadurch **gekennzeichnet**, daß das Schallwellenausbreitungsmedium Wasser (20) ist.

5. Gerät nach Anspruch 1 dadurch **gekennzeichnet**, daß das Schallwellentherapiegerät außerdem einen Drehmomentsensor (56) aufweist, der an dem Antriebsmechanismus (36, 38, 40, 50, 60) angeordnet ist, um das auf den Antriebsmechanismus einwirkende Drehmoment zu erfassen, und einen Anschlagmechanismus zum Anhalten der Betätigung des Antriebsmechanismus (36, 38, 40, 50, 60) dann, wenn das Ausgangssignal des Drehmomentsensors (56) einen vorbestimmten Pegel übersteigt.

6. Gerät nach Anspruch 1 dadurch **gekennzeichnet**, daß das Schallwellentherapiegerät aufweist:
eine Rückzieheinrichtung (60), die auf der anderen Seite des Halters (14) angeordnet ist, um das Stabelement (32) dann zurückzuziehen, wenn der Endabschnitt des Stabelements (32) von der Oberfläche des Patienten über das Sackteil (22-1, 22-2) eine vorbestimmte Aufwärtskraft empfängt.

7. Gerät nach Anspruch 6 dadurch **gekennzeichnet**, daß die Rückzieheinrichtung (60) ein Torsionsfedermechanismus (62) ist, der an dem Antriebsmechanismus (36, 38, 40, 50, 60) gelagert ist.

## Revendications

1. Appareil thérapeutique à ondes acoustiques, comprenant :
un support (14) présentant une surface concave au moins d'un côté et ayant également un trou passant en sa partie centrale;
un moyen (10, 12) générateur d'ondes acoustiques, qui est placé dudit côté dudit support (14) et sert à produire des ondes acoustiques en vue d'un traitement médical ;
un élément formant un sac (22-1, 22-2), disposé dudit côté du support (14) et conçu pour être mis en contact avec une surface (26) d'un patient, et contenant un milieu de propagation d'ondes acoustiques ;
un élément formant une tige (32), inséré dans ledit trou passant du support (14) et conçu pour pouvoir être déplacé verticalement dans ledit élément sac (22-1, 22-2); et
une sonde à ultrasons (34) destinée à former des images tomographiques d'ondes ultrasonores, qui est placée à la partie terminale de l'élément tige (32) se trouvant du côté de l'élément sac;
caractérisé en ce que ledit appareil thérapeutique à ondes acoustiques comprend :
un mécanisme d'entraînement (36, 38, 40, 50, 60) servant à déplacer verticalement la sonde à ultrasons (34) par rapport au support (14) ; et
un moyen (52 ; 52A) de limitation de couple, qui est prévu sur ledit mécanisme d'entraînement (36, 38, 40, 50, 60) afin de limiter à une valeur prédéterminée la force de pression avec laquelle l'élément tige (32) est appliqué sur la surface (26).

2. Appareil selon la revendication 1, caractérisé en ce que ledit moyen générateur d'ondes acoustiques (10,12) produit des ondes sous forme d'impulsions intenses.

3. Appareil selon la revendication 1, caractérisé en ce que ledit moyen générateur d'ondes acoustiques (10, 12) produit des ondes continues intenses.

4. Appareil selon la revendication 1, caractérisé en ce que ledit milieu de propagation d'ondes acoustiques est l'eau (20).

5. Appareil selon la revendication 1, caractérisé en ce qu'il comprend en outre un détecteur de couple (56) placé sur ledit mécanisme d'entraînement (36, 38, 40, 50, 60) afin de détecter le couple agissant sur le mécanisme d'entraînement et un mécanisme d'arrêt servant à arrêter l'entraînement dudit mécanisme d'entraînement (36, 38, 40, 50, 60) lorsque le signal de sortie du détecteur de couple (56) dépasse un niveau prédéterminé.

6. Appareil selon la revendication 1, caractérisé en ce qu'il comprend un moyen de retrait (60) disposé de l'autre côté du support (14) et servant à effectuer le retrait dudit élément tige (32) lorsque le partie terminale de l'élément tige (32) reçoit une force prédéterminée dirigée vers le haut de la part de la surface du patient via l'élément sac (22-1, 22-2).

7. Appareil selon la revendication 6, caractérisé en ce que ledit moyen de retrait (60) est un mécanisme à ressort de torsion (62) monté sur ledit mécanisme d'entraînement (36, 38, 40, 50, 60).
